# EUROPEAN PATENT SPECIFICATION

(11) **EP 1 617 899 B1**
(45) Date of publication and mention of the grant of the patent: **22.06.2011**
(21) Application number: 04750168.9
(22) Date of filing: 14.04.2004
(51) Int. Cl.: A61N 1/362

(54) **SUSTAINED PACING TO PREVENT ATRIAL FIBRILLATION**
UNTERSTÜTZTE HERZSTIMULATION ZUM VERHINDERN VON ATRIALER FIBRILLATION
PREVENTION DE LA FIBRILLATION AURICULAIRE PAR ENTRAINEMENT PROLONGE

(30) Priority: 24.04.2003 US 423035
(43) Date of publication of application: 25.01.2006
(73) Proprietor: Medtronic, Inc., Minneapolis, MN 55432-5604 (US)
(72) Inventor: VAN DALEN, Bert, T. F. C., NL-3845 BH Harderwijk (NL)
(74) Representative: Hughes, Andrea Michelle
(86) International application number: PCT/US2004/011648
(87) International publication number: WO 2004/096351

(56) References cited:
- US-A1- 2003 009 198
- US-A1- 2003 032 986

## Description

The invention relates to implantable medical devices and, more particularly, to cardiac pacemakers that deliver pacing pulses in response to a premature atrial contraction to prevent atrial fibrillation.

Tachyarrhythmias are episodes of high-rate cardiac depolarizations. Tachyarrhythmias may occur in one chamber of the heart or may be propagated from one chamber to another. Some tachyarrhythmias are sufficiently high in rate to compromise cardiac output from the chamber affected, leading to loss of consciousness or death in the case of ventricular fibrillation, or weakness and dizziness in the case of atrial fibrillation. Atrial fibrillation is often debilitating, due to the loss of atrial cardiac output, and may sometimes lead to ventricular fibrillation.

Fibrillation may be terminated by administering high energy level cardioversion or defibrillation shocks until the fibrillation is terminated. For example, an implanted device may deliver defibrillation shocks via an electrode carried by a lead implanted within the heart. Unfortunately, the high energy levels associated with cardioversion/defibrillation shocks can cause significant pain to the patient. In addition, atrial defibrillation shocks can sometimes give rise to ventricular arrhythmias. Therefore, it is generally desirable to avoid the onset of atrial fibrillation, and the need to apply defibrillation shocks. Some implanted devices deliver anti-tachycardia pacing pulses to terminate detected episodes of atrial tachycardia. Other devices are configured to predict the onset of atrial fibrillation, and deliver pacing pulses to prevent the atrial fibrillation from occurring. In particular, a device may be configured to detect premature atrial contractions (PACs) as trigger events that indicate the onset of atrial fibrillation. Delivery of pacing pulses in response to PAC detection can help prevent or decrease the occurrence of atrial fibrillation. Pacing pulses delivered in response to PAC detection are sometimes referred to as post-PAC pacing pulses.

US 2003/032986, accordingly to the preamble of claim 1, teaches a method and system for terminating atrial fibrillation by inducing ventricular extra-systole. US 2003/0009891 discloses a method and system for preventing atrial fibrillation by rapid pacing intervention.

The invention provides an implantable medical device as defined in claim 1, and preferably comprising an implanted cardiac lead carrying a sense electrode to sense a premature contraction, a pulse generator that delivers a sequence of pacing pulses in response to detection of a premature condition, and a controller that determines a number of the pacing pulses in the sequence based on efficacy of a previously delivered sequence of pacing pulses in preventing atrial fibrillation. The device can deliver a sustained sequence of pacing pulses in response to a premature atrial contraction (PAC) to prevent atrial fibrillation or reduce atrial fibrillation burden. In accordance with the preferred embodiment, the number of pacing pulses is sustained for an extended period of time to more effectively stabilize the atrium with the aid of the cardiac memory phenomenon. The number of pacing pulses delivered in a given post-PAC pacing sequence can be determined based on the efficacy of a prior post-PAC pacing sequence in preventing atrial fibrillation. In some embodiments, the post-PAC pacing interval also may be adjusted based on the efficacy of a prior post-PAC pacing sequence in preventing atrial fibrillation.

If a prior post-PAC pacing sequence was ineffective in preventing a previous episode of atrial fibrillation, the number of pulses delivered in the next post-PAC pacing sequence may be increased. The number of pulses may be incrementally increased in a step-up manner over a series of post-PAC pacing sequences in an effort to deliver a sustained number of pulses that is successful in preventing atrial fibrillation. In this manner, the pacing technique may arrive at a number of pacing pulses sufficient to take advantage of the cardiac memory phenomenon and thereby stabilize the atrium and improve the post-PAC response.

Once an effective number of pulses is determined and found to be successful, however, the number of pulses in subsequent post-PAC pacing sequences may be incrementally decreased in a step-down manner to seek a threshold number of pulses that are effective in preventing atrial fibrillation. This adaptive approach is useful in determining an effective number of post-PAC pacing pulses at a particular time, while reducing the overall number of pulses delivered to the patient over time. In particular, the post-PAC response of the atrium may vary with time and patient state. A step-up process of seeking an effective number of post-PAC pacing pulses followed by a step-down process to seek a threshold number of pulses can enhance the efficacy of the post-PAC pacing sequence in preventing atrial fibrillation.

In conjunction with the determination of the number of pulses, the pacing interval also may be determined on a selective basis based on the efficacy of previously applied post-PAC sequences. If a previous post-PAC sequence was ineffective in preventing atrial fibrillation, for example, the pacing interval may be incremental reduced in subsequent post-PAC sequences to seek an effective pacing interval. Alternatively, the pacing interval may be increased in the event previous sequences have been routinely effective, thereby reducing the number of paces delivered to the patient.

Preferred embodiments will now be described by way of example only with reference to the drawings.
FIG. 1 is a schematic view of an exemplary implantable medical device implanted within a human body.
FIG. 2 is a diagram of the implantable medical device of FIG. 1 located in and near a heart.
FIG. 3 is a block diagram illustrating the constituent components of the implantable medical device depicted in FIGS. 1 and 2.
FIG. 4 is a flow diagram illustrating a technique for delivery of post-PAC pacing in accordance with an embodiment of the invention.
FIG. 5 is a flow diagram illustrating a technique for delivery of post-PAC pacing in accordance with another embodiment of the invention.
FIG. 6 is a flow diagram illustrating adjustment of the number of post-PAC pacing pulses delivered to the atrium.
FIG. 7 is a flow diagram illustrating adjustment of the pacing interval of post-PAC pacing pulses delivered to the atrium.

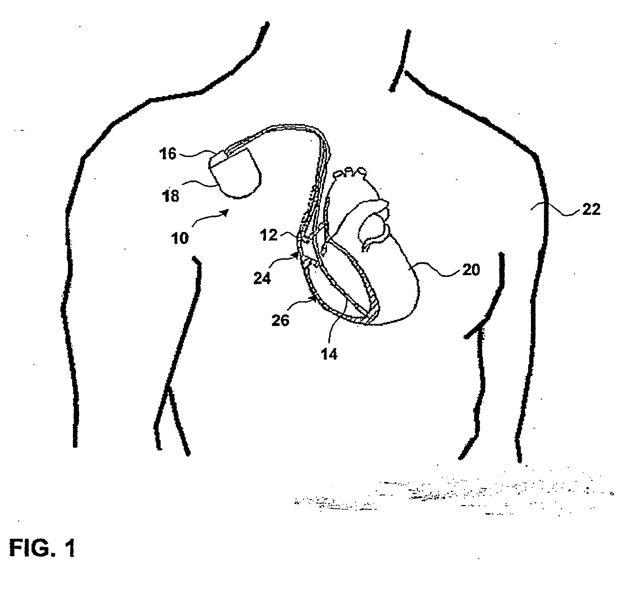

FIG. 1 is a schematic view of an exemplary implantable medical device 10 implanted within a human patient 22. IMD 10 detects premature atrial contractions (PACs), and delivers post-PAC pacing pulse sequences to prevent atrial fibrillation. In accordance with the invention, IMD 10 may be configured to apply a sustained number of post-PAC pacing pulses to take advantage of the cardiac memory phenomenon by which myocardial cells tend to be conditioned by prolonged application of pacing stimuli. For example, IMD 10 may apply greater than two post-PAC pacing pulses and, in some embodiments, eight or more post-PAC pacing pulses.

IMD 10 also may adjust the number of post-PAC pacing pulses delivered to the heart based on the efficacy of previously delivered post-PAC pacing sequences in preventing atrial fibrillation. In addition, IMD 10 may be configured to adjust the post-PAC pacing interval based on the efficacy of previously delivered post-PAC pacing sequences. In this manner, continuous monitoring can be used to permit the use of a lesser number of paces when possible, and create a dynamic post-PAC pacing behavior tailored to individual patient situations over time. These features will be described in greater detail below.

In the example of FIG. 1, IMD 10 is a pacemaker comprising atrial pacing and sensing lead 12 and ventricular pacing and sensing lead 14 attached to connector module 16 of hermetically sealed enclosure 18 and implanted near human or mammalian heart 20 of patient 22. Pacing and sensing leads 12 and 14 sense electrical signals attendant to the depolarization and depolarization of the heart 20, and further provide pacing pulses for causing depolarization of cardiac tissue in the vicinity of the distal ends thereof. Leads 12 and 14 may have unipolar or bipolar electrodes disposed thereon.

IMD 10 is one example of a device capable of practicing the invention, in that IMD 10 has the capability of detecting PACs, and pacing the right atrium in response to the detected PAC in an attempt to prevent the onset of atrial fibrillation. In particular, atrial pacing and sensing lead 12 senses activation of the right atrium 24, and can deliver post-PAC pacing pulses to right atrium 24.

Ventricular pacing and sensing lead 14 senses activation of the right ventricle 26, and can pace right ventricle 26. IMD 10 is not the only implantable medical device that may practice the invention, however. The invention alternatively may be practiced by implantable medical devices that are configured to pace three or four chambers of heart 20, and that provide atrioventricular synchrony.

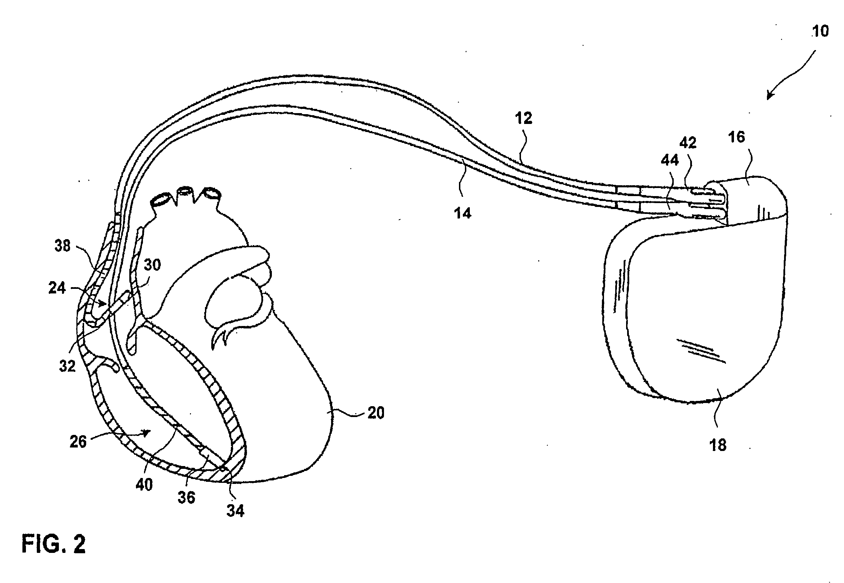

FIG. 2 is a diagram of implantable medical device 10 of FIG. 1 located in and near heart 20. FIG. 2 shows IMD 10, with connector module 16 and hermetically sealed enclosure 18. Atrial and ventricular pacing leads 12 and 14 extend from connector module 16 to the right atrium 24 and right ventricle 26, respectively, of heart 20. Atrial electrodes 30 and 32 disposed at the distal end of atrial pacing lead 12 are located in right atrium 24. Ventricular electrodes 34 and 36 disposed at the distal end of ventricular pacing lead 14 are located in right ventricle 26.

Pulse generators (not shown in FIG. 2) inside enclosure 18 generate pacing pulses. The pacing pulses are delivered to right atrium 24 or right ventricle 26 by electrodes 30, 32, 34, 36. In accordance with the invention, a selected number of post-PAC pacing pulses are applied in a sequence to right atrium 24 to prevent atrial fibrillation. A processor (not shown in FIG. 2) in IMD 10 responds to detected PACs by directing delivery of post-PAC pacing pulses to prevent atrial fibrillation and thereby maintain effective hemodynamic function within heart 20.

In addition to pacing, IMD 10 may apply other forms of therapy. In FIG. 2, for example, atrial lead 12 and ventricular lead 14 include defibrillation electrodes 38 and 40, respectively. Defibrillation electrodes 38 and 40 deliver defibrillation shocks to right atrium 24 or right ventricle 26 when necessary to terminate an episode of atrial or ventricular defibrillation. Atrial and ventricular leads 12, 14 each include an elongated insulative lead body carrying one or more conductors insulatively separated from one another. At the proximal end of leads 12, 14 are bifurcated connectors 42, 44, which electrically couple the connectors to connector module 16 of IMD 10.

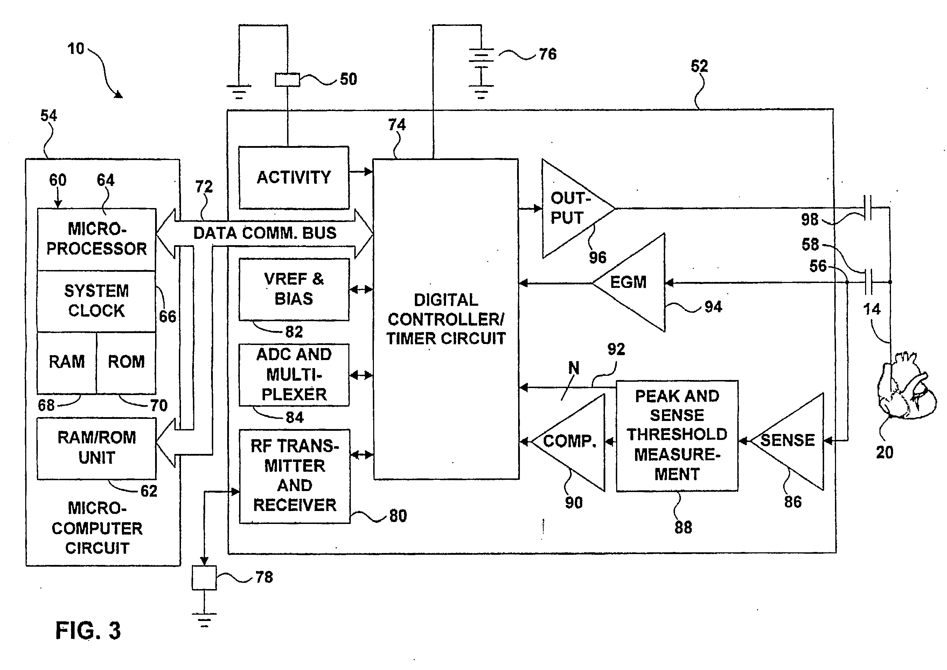

FIG. 3 shows a block diagram illustrating exemplary components of IMD 10 in accordance with one embodiment of the invention, in which IMD 10 is a pacemaker having a microprocessor-based architecture. As shown in FIG. 3, IMD 10 includes one or more activity sensors 50. Activity sensor 50 may include an accelerometer, such as a piezoceramic accelerometer or a microelectromechanical accelerometer, that provides a sensor output that varies as a function of a measured parameter relating to a patient's metabolic requirements. In other words, activity sensor 50 detects motion of patient 22 that accompanies physical activity, and may adjust the pacing rate to the metabolic needs associated with the physical activity.

The output of activity sensor 50 is coupled to input/output circuit 52. Input/ouput circuit 52 contains analog circuits for interfacing with heart 20, activity sensor 50, and other components and circuits for the application of stimulating pulses to heart 20. For ease of illustration, IMD 10 in FIG. 3 is shown with only lead 14 connected. Similar circuitry and connections not explicitly shown in FIG. 3 apply to lead 12 (shown in FIGS. 1 and 2), however. Lead 14 is coupled to node 56 in IMD 10 through input capacitor 58.

The rate of heart 20 is controlled by software-implemented algorithms stored within microcomputer circuit 54. Microcomputer circuit 54 comprises on-board circuit 60 and off-board circuit 62. On-board circuit 60 preferably includes microprocessor 64, system clock circuit 66 and on-board random access memory (RAM) 68 and read-only memory (ROM) 70. Off-board circuit 62 comprises a RAM/ROM unit. On-board circuit 60 and off-board circuit 62 are each coupled by data communication bus 72 to digital controller/timer circuit 74. Microcomputer circuit 54 may comprise a custom integrated circuit device augmented by standard RAM/ROM components.

Microcomputer circuit 54 is an example of a processor that directs delivery of post-PAC paces in response to a detected PAC to prevent atrial fibrillation and thereby maintain hemodynamic performance. In accordance with the invention, IMD 10 detects premature atrial contractions (PACs), and delivers post-PAC pacing sequences to prevent atrial fibrillation.

IMD 10 is configured to adjust the number of post-PAC pacing pulses delivered to the heart in response to PAC detection based on the efficacy of previously delivered post-PAC pacing sequences in preventing atrial fibrillation. In addition, IMD 10 may be configured to adjust the post-PAC pacing interval based on the efficacy of prior post-PAC pacing sequences. In this manner, IMD 10 may be more effective in preventing atrial fibrillation, and thereby reducing atrial fibrillation burden and improving hemodynamic performance.

If a prior post-PAC pacing sequence was ineffective in preventing an episode of atrial fibrillation, for example, IMD 10 may increase the number of pulses in a subsequent post-PAC pacing sequence. IMD 10 may incrementally increase the number of pulses in a step-up manner over a series of post-PAC pacing sequences in an effort to deliver a number of pulses that is successful in preventing atrial fibrillation. In this manner, IMD 10 may arrive at a sustained number of pacing pulses sufficient to take advantage of the cardiac memory phenomenon, and thereby stabilize the atrium and improve the post-PAC response.

In general, a sustained number of pacing pulses may refer to a post-PAC pacing sequence in excess of two pacing pulses. However, an increased number of pacing pulses may be more effective in exploiting the cardiac memory phenomenon. As one example, IMD 10 may start by applying eight or more post-PAC pacing pulses and then adjust the number of pacing pulses in subsequent post-PAC sequences based on the efficacy or prior sequences.

Also, a sustained number of post-PAC pulses sufficient to prevent atrial fibrillation may vary among different patients, different times, different conditions, and different disease states. As one illustration, the number of post-PAC pulses needed to prevent atrial fibrillation may vary according to intake of medication by the patient. As another example, a given patient may present different responses with varying levels of physical activity. For these reasons, IMD 10 operates in an adaptive manner to seek an effective number of pacing pulses.

Once IMD 10 determines an effective number of pulses that is found to be successful, the number of pulses in subsequent post-PAC pacing sequences may be incrementally decreased in a step-down manner to seek a threshold number of pulses that are effective in preventing atrial fibrillation. In this manner, IMD 10 may arrive at a number of pacing pulses sufficient to take advantage of the cardiac memory phenomenon and thereby stabilize the atrium and improve the post-PAC response.

Once an effective number of pulses is determined and found to be successful, however, IMD 10 may incrementally decrease the number of pulses in subsequent post-PAC pacing sequences in a step-down manner to seek a threshold number of pulses that are effective in preventing atrial fibrillation. This adaptive approach is useful in determining an effective number of post-PAC pacing pulses at a particular time, while reducing the overall number of pulses delivered to the patient over time. In particular, the post-PAC response of right atrium 24 may vary with time and patient state. A step-up process of seeking an effective number of post-PAC pacing pulses followed by a step-down process to seek a threshold number of pulses can enhance the efficacy of the post-PAC pacing sequence in preventing atrial fibrillation.

In conjunction with the determination of the number of pulses, IMD 10 also may determine the pacing interval of the post-PAC pacing pulses, i.e., the time between successive post-PAC pacing pulses, on a selective basis based on the efficacy of previously applied post-PAC sequences. If a previous post-PAC sequence was ineffective in preventing atrial fibrillation, for example, IMD 10 may incrementally reduce the pacing interval in subsequent post-PAC sequences to seek an effective pacing interval. Alternatively, IMD 10 may increase the pacing interval in the event previous sequences have been routinely effective, thereby reducing the number of paces delivered to the patient.

Again, IMD 10 may adjust the number of pacing pulses in view of the efficacy of a previous post-PAC pacing sequence. In addition, IMD 10 may monitor the effect of sustained post-PAC response against the atrial fibrillation burden, the number of fibrillation episodes, or the time to the next episode and increase the number of paces in the post-PAC pacing sequence. Accordingly, algorithms for adjustment of the number of paces may range from simple to complex, and may take into account the immediately preceding post-PAC pacing sequence and atrial fibrillation episode or the effects of multiple sequences and episodes over a period of time.

Electrical components shown in FIG. 3 are powered by an appropriate implantable battery power source 76. For ease of illustration, the coupling of battery power to the various components of IMD 10 is not shown in FIG. 3. Antenna 78 is connected to input/output circuit 52 to permit uplink/downlink telemetry through radio frequency (RF) transmitter and receiver telemetry unit 80. IMD 10 in FIG. 3 is programmable by an external programming unit (not shown in the figures) that communicates with IMD 10 via antenna 78 and RF transmitter and receiver telemetry unit 80.

VREF and Bias circuit 82 generates stable voltage reference and bias currents for analog circuits included in input/output circuit 52. Analog-to-digital converter (ADC) and multiplexer unit 84 digitizes analog signals and voltages to provide "real-time" telemetry intracardiac signals and battery end-of-life (EOL) replacement functions.

Operating commands for controlling the timing of IMD 10 are coupled from microprocessor 64 via data bus 72 to digital controller/timer circuit 74, where digital timers and counters establish the overall escape interval of the IMD 10 as well as various refractory, blanking and other timing windows for controlling the operation of peripheral components disposed within input/output circuit 52.

Digital controller/timer circuit 74 is coupled to sensing circuitry, including sense amplifier 86, peak sense and threshold measurement unit 88 and comparator/threshold detector 90. Sense amplifier 86 amplifies electrical cardiac signals sensed via lead 14 and provides an amplified signal to peak sense and threshold measurement circuitry 88, which in turn provides an indication of peak sensed voltages and measured sense amplifier threshold voltages on multiple conductor signal path 92 to digital controller/timer circuit 74. An amplified sense amplifier signal is also provided to comparator/threshold detector 90.

Digital controller/timer circuit 74 is further coupled to electrogram (EGM) amplifier 94 for receiving amplified and processed signals sensed by lead 14. The electrogram signal provided by EGM amplifier 94 is employed, for example, when IMD 10 is being interrogated by an external programmer to transmit a representation of a cardiac analog electrogram. Output pulse generator 96 provides pacing stimuli to heart 20 through coupling capacitor 98 in response to a pacing trigger signal provided by digital controller/timer circuit 74.

IMD 10 may sense the P-waves, i.e., atrial depolarizations, via lead 12, sense amplifier 86, peak sense and threshold measurement unit 88 and comparator/threshold detector 90. The time interval of the P-wave relative to a previous P-wave, i.e., the coupling interval, can be used to detect PACs.

Consequently, sense amplifier 86, peak sense and threshold measurement unit 88 and comparator/threshold detector 90 may be configured to serve as PAC detector. In response to PAC detection, digital controller/timer circuit 74 controls delivery of a sequence of post-PAC pacing pulses. The number of pulses in the post-PAC sequence may be determined by microprocessor 64.

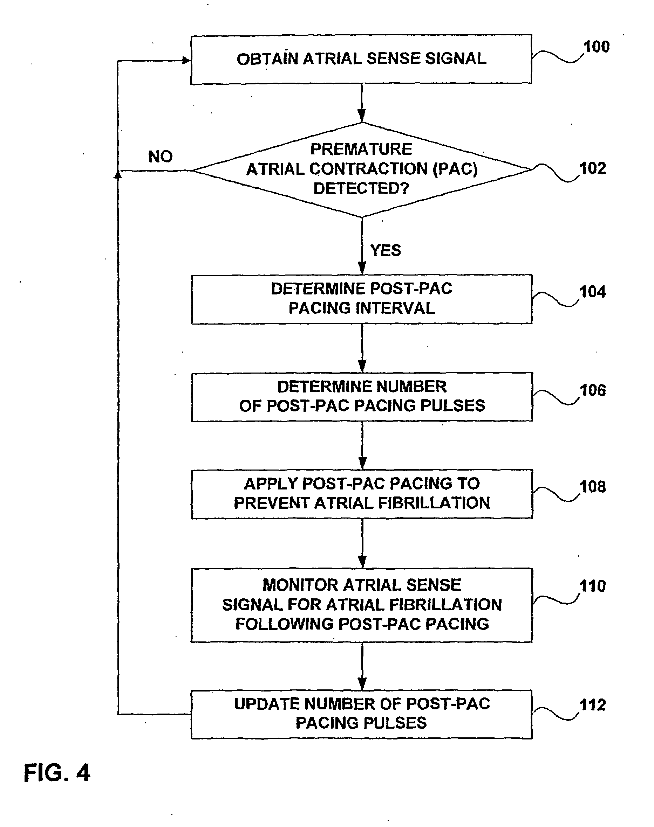

FIG. 4 is a flow diagram illustrating a technique for delivery of post-PAC pacing in accordance with the invention. FIG. 4 is a flow diagram illustrating techniques that may be performed by IMD 10 in accordance with one or more embodiments of the invention.
As shown in FIG. 4, IMD 10 obtains an atrial sense signal (100), e.g., via atrial sense lead 12, and analyzes the signal to detect whether a PAC has occurred (102).

For example, IMD 10 may compare the interval between successive P-waves to a threshold to detect a PAC. If no PAC is detected, IMD 10 continues to obtain atrial sense signals (100). If a PAC is detected, however, IMD 10 recognizes a trigger event that may be indicative of the onset of atrial fibrillation.

In an effort to prevent atrial fibrillation, and the resulting hemodynamic burden to patient 12, IMD 10 delivers a post-PAC sequence of pacing pulses. In accordance with the invention, IMD 10 determines a post-PAC pacing interval (104), i.e., the interval between successive pacing pulses in the post-PAC sequence. The post-PAC pacing interval may be determined as a function of the coupling interval. For example, the post-PAC pacing interval may be a fixed percentage of the coupling interval. Alternatively, the post-PAC pacing interval may be adaptive, in accordance with an embodiment of the invention.

As further shown in FIG. 4, IMD 10 determines the number of post-PAC pacing pulses in the sequence (106). To exploit the cardiac memory phenomenon, the post-PAC pacing sequence may begin with an elevated number of pacing pulses. For example, the post-PAC pacing sequence may include eight or more pacing pulses.
IMD 10 applies the post-PAC pacing sequence to right atrium 24 to prevent occurrence of atrial fibrillation (108). Upon delivery of the post-PAC pacing sequence, IMD 10 monitors the atrial sense signal for atrial fibrillation (110) to determine whether the post-PAC pacing sequence was successful in preventing atrial fibrillation. Based on the efficacy of the prior post-PAC pacing sequence, IMD 10 updates the number of post-PAC pacing pulses to be used in the next post-PAC pacing sequence.

If the prior post-PAC pacing sequence was successful in preventing atrial fibrillation, IMD 10 reduces the number of pacing pulses in the post-PAC pacing sequence to seek a threshold number of pacing pulses sufficient to prevent atrial fibrillation.

This step-down process may proceed in an iterative fashion over a series of separate PAC detections until a number of pacing pulses that are ineffective in preventing atrial fibrillation is determined. Then, the number of pacing pulses may be incrementally increased upward so that the number exceeds the threshold for effective prevention of atrial fibrillation.

If the prior post-PAC was not successful in preventing atrial fibrillation, IMD 10 may increase the number of pacing pulses in the next post-PAC pacing sequence to seek a threshold number of pacing pulses sufficient to prevent atrial fibrillation. Like the step-down process described above, this step-up process may proceed in an iterative fashion over a series of separate PAC detections until a number of pacing pulses that are effective in preventing atrial fibrillation is determined.

For example, the number of post-PAC pacing pulses may be increased in linear or non-linear increments, e.g., from 8 pacing pulses up to approximately 100 paces, to provide a sustained post-PAC sequence. The increased number of post-PAC pacing pulses can take advantage of the cardiac memory phenomenon by which myocardial cells tend to become conditioned to pacing stimuli. By sustaining the post-PAC pacing over an increased number of pacing pulses, the pacing sequence may be more effective in preventing atrial fibrillation.

Periodically, if the determined number of post-PAC pulses is virtually always effective in preventing atrial fibrillation, or effective a high percentage of the time, the number of post-PAC pacing pulses may again be decreased to seek a minimum threshold number sufficient to prevent atrial fibrillation.

IMD 10 may monitor the effect of sustained post-PAC response against the atrial fibrillation burden, the number of fibrillation episodes, or the time to the next episode and increase the number of paces in the post-PAC pacing sequence. In this manner, IMD 10 may benefit from collection of episodic information. In some embodiments, the amount by which the number of pacing pulses is increased may be determined as a function of such information.

For example, the fibrillation burden, number of fibrillation episodes, or time to the next episode over a period of time may serve as indices for selection of a predetermined number of pacing pulses. Other indices may include intake of medication at a particular time of day, activity level, or the like. The number of pacing pulses may be predetermined and prestored based on the collected episodic information. The result is a dynamic post-PAC pacing algorithm that is better tailored to the individual patient situation over time.

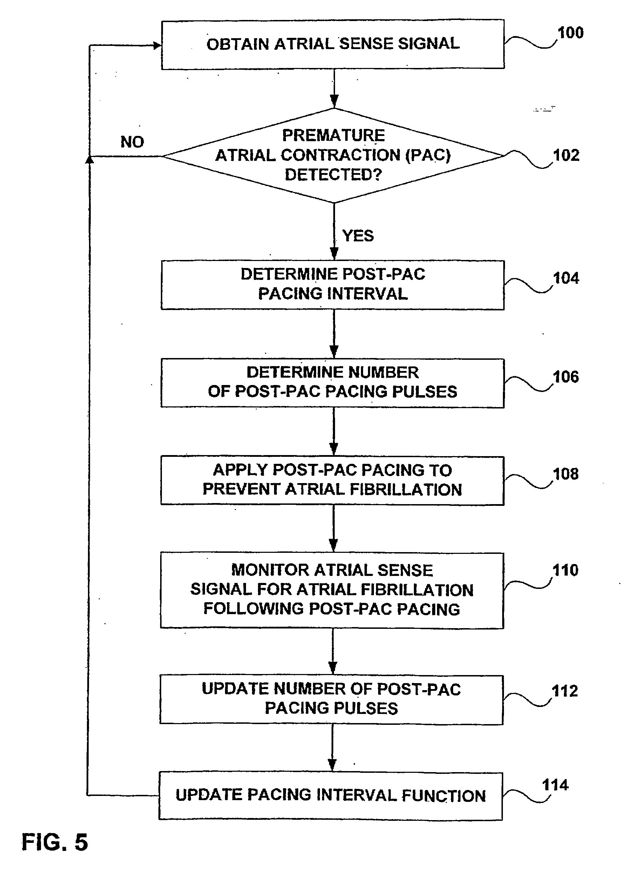

Hence, the process may involve step-up and step-down progressions over time, and select different numbers of post-PAC pacing pulses appropriate for different times or patient conditions. The post-PAC pacing provided by IMD 10 is adaptive, and can be more efficient in terms of the number of pacing pulses delivered to the patient over time. FIG. 5 is a flow diagram illustrating a technique for delivery of post-PAC pacing in accordance with another embodiment of the invention. The technique shown in FIG. 5 corresponds generally to that of FIG. 4, but further depicts updating of a pacing interval function (114) used to determine a post-PAC pacing interval (104) by IMD 10. The post-PAC pacing interval is adjusted according to an pacing interval function.

The pacing interval function may specify the post-PAC pacing interval as a percentage of the PAC coupling interval, i.e., the interval between the PAC and the contraction preceding the PAC. Rather than a fixed percentage pacing interval function, however, IMD 10 updates the pacing interval function based on the efficacy of a prior post-PAC pacing sequence that used a given pacing interval.

More specifically, IMD 10 monitors the atrial sense signal for atrial fibrillation following the prior post-PAC pacing sequence (110) to determine whether the prior sequence was successful. If the prior post-PAC pacing sequence was not successful in preventing atrial fibrillation, IMD 10 may update the pacing interval function (114) to reduce the post-PAC pacing interval.

For example, IMD 10 may reduce a percentage associated with the pacing interval function such that the pacing interval for the next post-PAC pacing sequence is shorter than the pacing interval for the prior post-PAC pacing sequence. In this manner, IMD 10 is able to adapt the post-PAC pacing interval to seek intervals that may be more effective in preventing atrial fibrillation.

The post-PAC pacing interval may be increased or decreased based on the efficacy of prior post-PAC pacing sequences. Like the number of post-PAC pacing pulses, the pacing interval can be incrementally increased and decreased in step-up and step-down modes to achieve threshold pacing intervals that are effective in preventing atrial fibrillation but tend to avoid delivery of an excessive number of pacing pulses. Also, adjustment of the post-PAC pacing interval, as shown in FIG. 5, may be combined with adjustment of the number of post-PAC pacing pulses. In this manner, IMD 10 seeks more effective post-PAC pacing sequence.

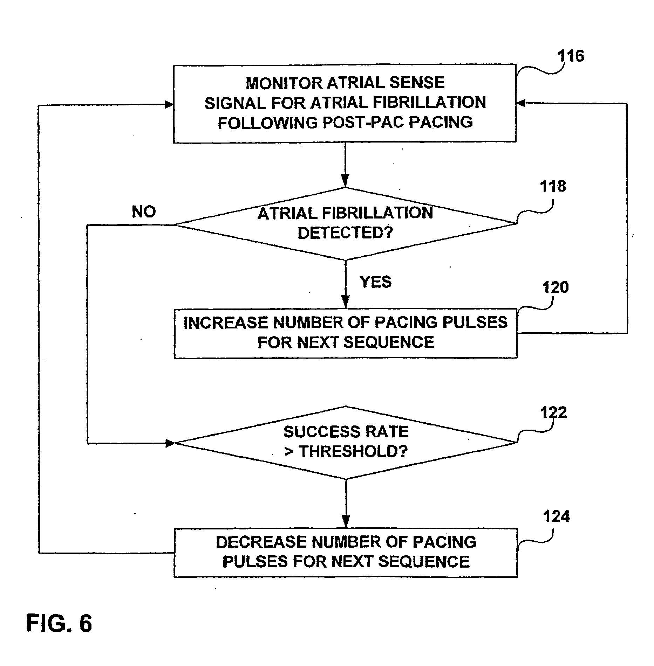

FIG. 6 is a flow diagram illustrating adjustment of the number of post-PAC pacing pulses delivered to the atrium. In general, FIG. 6 depicts step-up and step-down modes for arriving at a threshold number of post-PAC pacing pulses that tends toward a minimum number of pulses sufficient to prevent atrial fibrillation. As shown in FIG. 6, IMD 10 monitors the atrial sense signal for atrial fibrillation following delivery of post-PAC pacing sequence (116).

If atrial fibrillation is detected (118), IMD 10 increases the number of pacing pulses delivered for the next sequence (120). By increasing the number of pacing pulses, IMD 10 seeks a number of pacing pulses sufficient to prevent atrial fibrillation. If atrial fibrillation is not detected (118), and the rate of success for the present number of post-PAC pacing pulses exceeds a threshold (122), IMD 10 decreases the number of pacing pulses for the next sequence (124).

If the number of pacing pulses used in a previous post-PAC pacing sequence has been used effectively over multiple PAC detections, it may be possible to seek a reduced number of paces that still offers effective prevention of atrial fibrillation. Thus, once an effective number of post-PAC pacing pulses has been determined, that number of pulses may be used repeatedly for subsequent PAC detections to assess continued effectiveness in preventing atrial fibrillation. If the repeated applications of the number of pulses yields a high success rate, IMD 10 then may initiate the step-down mode described herein to seek a reduced number of pulses that is still effective.

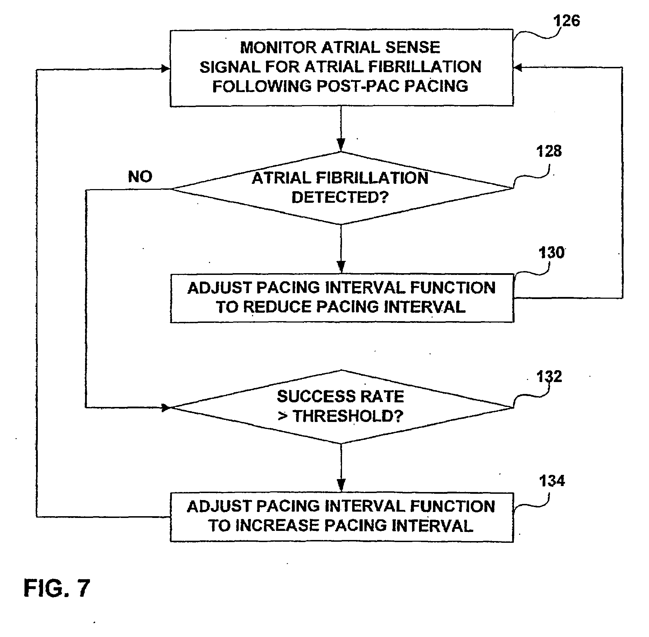

FIG. 7 is a flow diagram illustrating adjustment of the pacing interval of post-PAC pacing pulses delivered to the atrium. In general, FIG. 7 depicts step-up and step-down modes for arriving at a threshold post-PAC pacing interval that tends toward an optimum pacing interval for prevention of atrial fibrillation. As shown in FIG. 7, IMD 10 monitors the atrial sense signal for atrial fibrillation following delivery of post-PAC pacing sequence (126).

If atrial fibrillation is detected (118), IMD 10 adjusts the pacing interval function to reduce the pacing interval delivered for the next post-PAC pacing sequence (128). By reducing the pacing interval, IMD 10 seeks a pacing interval sufficient to prevent atrial fibrillation. If atrial fibrillation is not detected (130), and the rate of success for the present post-PAC pacing interval exceeds a threshold (132), IMD 10 adjusts the pacing interval function to increase the number of pacing pulses for the next sequence (124). Many embodiments of the invention have been described. Various modifications may be made without departing from the scope of the claims. For example, the invention is not limited to the particular implantable medical devices described above, but may be practiced by a wide variety of implantable medical devices.

In addition, the invention may be embodied as a computer-readable medium that includes instructions for causing a programmable processor to carry out the methods described above. A "computer-readable medium" includes but is not limited to read-only memory, Flash memory and a magnetic or optical storage medium. The instructions may be implemented as one or more software modules, which may be executed by themselves or in combination with other software.

## Claims

1. An implantable medical device comprising:
means (10) for delivering a sequence of pacing pulses in response to detection of a premature contraction; and
means (74) for determining a number of the pacing pulses in the sequence based on the efficacy of a previously delivered sequence of pacing pulses in preventing atrial fibrillation; **characterized in that** the determining means (74) decreases the number of the pacing pulses in the sequence relative to the number of pacing pulses in the previously delivered sequence if the previously delivered sequence was successful in preventing atrial fibrillation.

2. The device of claim 1, wherein the determining means increases the number of the pacing pulses in the sequence relative to the number of pacing pulses in the previously delivered sequence if the previously delivered sequence was not successful in preventing atrial fibrillation.

3. The device of claim 1 or 2, further comprising means for determining a pacing interval for the pacing pulses in the sequence.

4. The device of claim 3, further comprising means for determining the pacing interval based on efficacy of the previously delivered sequence of pacing pulses in preventing atrial fibrillation.

5. The device of claim 4, wherein the means for determining the pacing interval reduces the pacing interval relative to a pacing interval for the pacing pulses in the previously delivered sequence if the previously delivered sequence was not successful in preventing atrial fibrillation.

6. The device of claim 4, wherein the means for determining the pacing interval increases the pacing interval relative to a pacing interval for the pacing pulses in the previously delivered sequence if the previously delivered sequence was successful in preventing atrial fibrillation.

7. The device of any preceding claim, wherein the premature contraction is a premature atrial contraction.

8. The device of claim 7, further comprising means for delivering the pacing pulses to the right atrium.

9. The device of any preceding claim, wherein the determining means determines the number of pacing pulses in the sequence to be at least eight pacing pulses.

10. An implantable medical device as claimed in any preceding claim, further comprising:
an implanted cardiac lead (12) carrying a sense electrode (30, 32) to sense a premature contraction.

## Patentansprüche

1. Implantierbare medizinische Vorrichtung mit:
Mitteln (10) zur Abgabe einer Sequenz von Stimulationspulsen als Antwort auf die Bestimmung einer vorzeitigen Kontraktion;
Mitteln (74) zur Bestimmung einer Anzahl der Stimulationspulse in der Sequenz auf Grundlage der Wirksamkeit einer zuvor abgegebenen Sequenz von Stimulationspulsen bei der Verhinderung atrialer Fibrillation; **dadurch gekennzeichnet, dass** die Bestimmungsmittel (74) die Anzahl der Stimulationspulse in der Sequenz relativ zu der Anzahl von Stimulationspulsen in der zuvor abgegebenen Sequenz verringern, wenn die zuvor abgegebene Sequenz bei der Verhinderung atrialer Fibrillation erfolgreich war.

2. Vorrichtung nach Anspruch 1, bei der die Bestimmungsmittel die Anzahl der Stimulationspulse in der Sequenz relativ zu der Anzahl von Stimulationspulsen in der zuvor abgegebenen Sequenz erhöhen, wenn die zuvor abgegebene Sequenz bei der Verhinderung atrialer Fibrillation nicht erfolgreich war.

3. Vorrichtung nach Anspruch 1 oder 2, ferner mit Mitteln zur Bestimmung eines Stimulationsintervalls für die Stimulationspulse in der Sequenz.

4. Vorrichtung nach Anspruch 3, ferner mit Mitteln zur Bestimmung des Stimulationsintervalls auf Grundlage der Wirksamkeit der zuvor abgegebenen Sequenz von Stimulationspulsen bei der Verhinderung von atrialer Fibrillation.

5. Vorrichtung nach Anspruch 4, bei der die Mittel zur Bestimmung des Stimulationsintervalls das Stimulationsintervall relativ zu einem Stimulationsintervall für die Stimulationspulse in der zuvor abgegebenen Sequenz verringern, wenn die zuvor abgegebene Sequenz bei der Verhinderung atrialer Fibrillation nicht erfolgreich war.

6. Vorrichtung nach Anspruch 4, bei der die Mittel zur Bestimmung des Stimulationsintervalls das Stimulationsintervall relativ zu einem Stimulationsintervall für die Stimulationspulse in der zuvor abgegebenen Sequenz erhöhen, wenn die zuvor abgegebene Sequenz bei der Verhinderung atrialer Fibrillation nicht erfolgreich war.

7. Vorrichtung nach einem der vorstehenden Ansprüche, bei der die vorzeitige Kontraktion eine vorzeitige atriale Kontraktion ist.

8. Vorrichtung nach Anspruch 7, ferner mit Mitteln zur Abgabe der Stimulationspulse an das rechte Atrium.

9. Vorrichtung nach einem der vorstehenden Ansprüche, bei der die Bestimmungsmittel die Anzahl von Stimulationspulsen in der Sequenz derart bestimmen, dass sie wenigstens acht Stimulationspulse darstellen.

10. Implantierbare medizinische Vorrichtung nach einem der vorstehenden Ansprüche, ferner mit:
einer implantierten Herzleitung (12), die eine Erfassungselektrode (30, 32) zur Erfassung einer vorzeitigen Kontraktion trägt.

## Revendications

1. Dispositif médical implantable comportant :
des moyens (10) pour délivrer une séquence d'impulsions de stimulation en réponse à la détection d'une contraction prématurée ; et
des moyens (74) pour déterminer un nombre des impulsions de stimulation dans la séquence sur la base de l'efficacité d'une séquence d'impulsions de stimulation délivrée précédemment pour empêcher une fibrillation auriculaire ; **caractérisé en ce que** les moyens de détermination (74) diminuent le nombre des impulsions de stimulation dans la séquence par rapport au nombre d'impulsions de stimulation dans la séquence délivrée précédemment si la séquence délivrée précédemment a réussi à empêcher une fibrillation auriculaire.

2. Dispositif selon la revendication 1, dans lequel les moyens de détermination augmentent le nombre des impulsions de stimulation dans la séquence par rapport au nombre d'impulsions de stimulation dans la séquence précédemment délivrée si la séquence précédemment délivrée n'a pas réussi à empêcher une fibrillation auriculaire.

3. Dispositif selon la revendication 1 ou 2, comportant en outre des moyens pour déterminer un intervalle de stimulation pour les impulsions de stimulation dans la séquence.

4. Dispositif selon la revendication 3, comportant en outre des moyens pour déterminer l'intervalle de stimulation sur la base de l'efficacité de la séquence délivrée précédemment d'impulsions de stimulation pour empêcher une fibrillation auriculaire.

5. Dispositif selon la revendication 4, dans lequel les moyens pour déterminer l'intervalle de stimulation réduisent l'intervalle de stimulation par rapport à un intervalle de stimulation pour les impulsions de stimulation dans la séquence précédemment délivrée si la séquence précédemment délivrée n'a pas réussi à empêcher une fibrillation auriculaire.

6. Dispositif selon la revendication 4, dans lequel les moyens pour déterminer l'intervalle de stimulation augmentent l'intervalle de stimulation par rapport à un intervalle de stimulation pour les impulsions de stimulation dans la séquence précédemment délivrée si la séquence précédemment délivrée a réussi à empêcher une fibrillation auriculaire.

7. Dispositif selon l'une quelconque des revendications précédentes, dans lequel la contraction prématurée est une contraction auriculaire prématurée.

8. Dispositif selon la revendication 7, comportant en outre des moyens pour délivrer les impulsions de stimulation à l'atrium droit.

9. Dispositif selon l'une quelconque des revendications précédentes, dans lequel les moyens de détermination déterminent le nombre d'impulsions de stimulation dans la séquence comme étant égal à au moins huit impulsions de stimulation.

10. Dispositif médical implantable selon l'une quelconque des revendications précédentes, comportant en outre :
une dérivation cardiaque implantée (12) supportant une électrode de détection (30, 32) pour détecter une contraction prématurée.
